# EUROPEAN PATENT SPECIFICATION

(11) **EP 2 387 404 B1**
(45) Date of publication and mention of the grant of the patent: **25.06.2014**
(21) Application number: 10700938.3
(22) Date of filing: 07.01.2010
(51) Int. Cl.: A61K 31/519, A61K 31/53, A61K 45/06, A61K 31/195, A61K 31/506, A61K 31/5377, A61P 15/00

(54) **Sgc stimulators and activators in combination with pde5 inhbitors for the treatment of erectile dysfunction**
SGC-Stimulatoren und Aktivatoren in Kombination mit PDE5-Inhibitoren zur Behandlung von erektiler Dysfunktion
Stimulateurs et activateurs de sgc en combinaison avec des inhibiteurs pde5 dans le traitement du dysfonctionnement érectile

(30) Priority: 17.01.2009 EP 09000619; 17.02.2009 EP 09002177
(43) Date of publication of application: 23.11.2011
(73) Proprietor: Bayer Intellectual Property GmbH, 40789 Monheim (DE)
(72) Inventor: SANDNER, Peter, 42113 Wuppertal (DE); STASCH, Johannes-Peter, 42651 Solingen (DE); BÖTTCHER, Michael-Friedrich, 51515 Kürten (DE)
(74) Representative: BIP Patents
(86) International application number: PCT/EP2010/000029
(87) International publication number: WO 2010/081647

(56) References cited:
- WO-A1-2008/138483
- WO-A2-2008/124505
- DE TEJADA I S: "Therapeutic strategies for optimizing PDE-5 inhibitor therapy in patients with erectile dysfunction considered difficult or challenging to treat" June 2004 (2004-06), INTERNATIONAL JOURNAL OF IMPOTENCE RESEARCH 200406 GB, VOL. 16, NR. SUPPL. 1, PAGE(S) S40-S42 , XP002594933 ISSN: 0955-9930 page S40, column 2

## Description

The present invention relates to soluble guanylate cyclase (sGC) and to phosphodiesterases (PDEs) and the pharmacology of sGC stimulators, sGC activators and PDE inhibitors. More particularly, the invention relates to the use of sGC stimulators and sGC activators in combination with PDE5 inhibitors for preparation of medicaments for the treatment of male erectile dysfunction (MED) in particular for the MED treatment of difficult to treat patients and patients not or not fully responding to PDE5 inhibitors. The present invention is defined in the claims. Subject matter which is not encompassed by the scope of the claims does not form part of the invention.

### Background of the invention

The cyclic nucleotides, cyclic adenosine monophosphate (cAMP) and cyclic guanosine monophosphate (cGMP), were discovered decades ago and represent one of the most important second messenger pathway within cells. It is well established that the regulation of intra-cellular cGMP pools have substantial impact on physiology, and pathophysiology and is one basic principle of pharmacological intervention (Eugenov et al. 2006). Besides the treatment of cardiovascular, lung or CNS-disorders there is ample evidence that an increase in cGMP is a very effective treatment option for urological disorders as well (Sandner et al. 2007a). It was shown that PDE5 inhibitors could be useful for the treatment of symptomatic BPH which is characterized by Overactive Bladder (OAB) and Lower Urinary Tract Symptoms (LUTS) (Porst et al. 2007, Koehler and McVary 2008). But most strikingly and well established is the effect of cGMP on penile function.

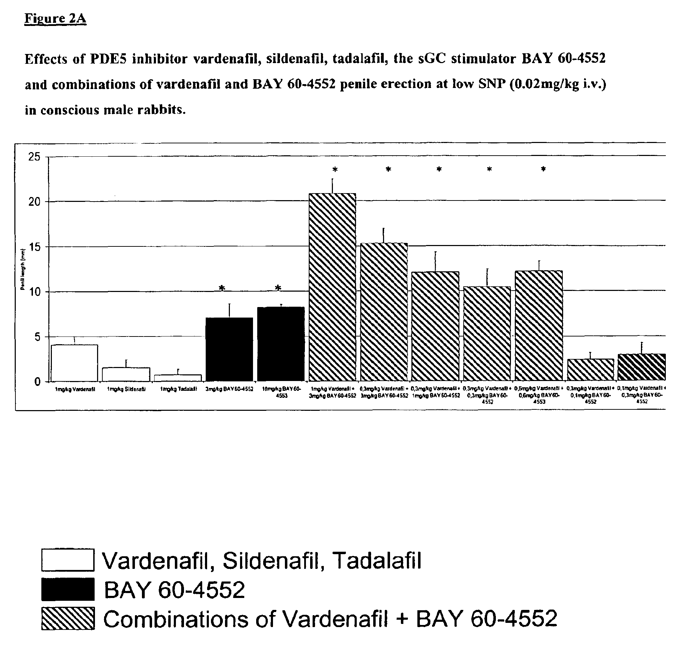

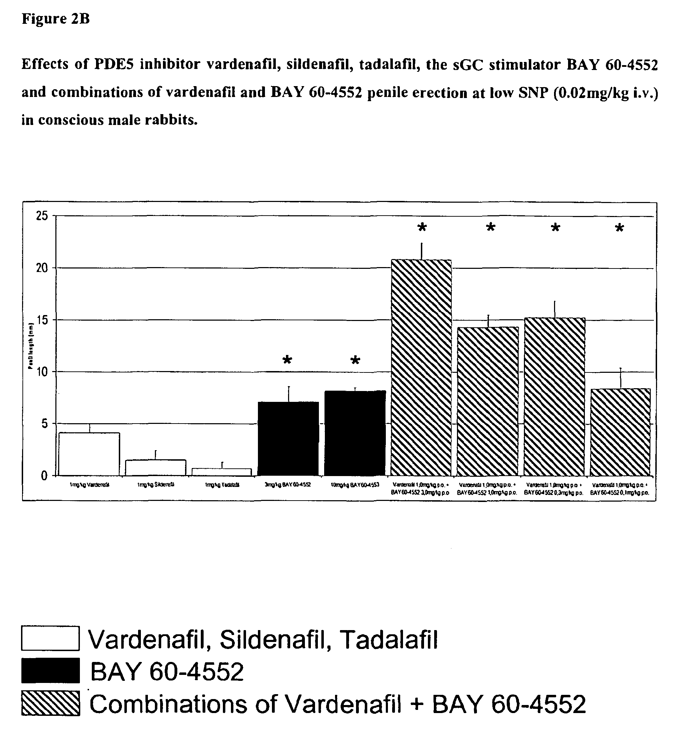

During recent years the basic understanding of penile function and erectile dysfunction (ED) has become very clear and is covered by variety of scientific publications and also broadly reviewed (De Tejada et al. 2004). Various neurotransmitters including NO, oxytocin and dopamine are recognized as pro-erectile neurotransmitters, necessary for penile erection. Nitric oxide, during either direct or psychogenic sexual stimulation, is synthesized by neuronal NO synthase (nNOS) in the nerve terminals of parasympathetic, non-adrenergic, non-cholinergic (NANC) neurons in the penis and also by endothelial NO synthase (eNOS) in the endothelial cells of the blood vessels and the lacunar spaces of the corpora cavernosa activates smooth muscle cell soluble guanylate cyclase (sGC). This NO-production results in increased intracellular cGMP levels, which leads to relaxation of smooth muscle in the corpus cavernosum and in penile arterioles. Relaxation of arterial smooth muscle is accompanied by increased blood flow to the penile corpora and an enlargement of the cavernosal tissue finally resulting penile erection. (Schultheiss and Stief 1999, Lue 2000) (figure 2). The level of cGMP is regulated by its rate of synthesis via guanylate cyclase (sGC) and its hydrolysis to the physiologically inactive GMP by the cGMP-hydrolyzing phosphodiesterases. PDE5 is the most prominent in the human corpus cavernosum and inhibition of PDE5 leads to an increase in the level of cGMP.

Thus the increase of cGMP levels via PDE5-inhibitors is the approved first line therapy and gold standard for the treatment of erectile dysfunction (Shabsigh et al. 2006, Sandner et al. 2007b).

However, the release of NO by NANC nerves and/or of the endothelium is impaired under pathological conditions such as diabetes and/or coronary heart disease, hypertension, or spinal cord injury, which consequently leads to reduced cGMP synthesis. Due to a limited endothelial or neuronal NO-production the efficacy of PDE5 inhibitors in these patients is limited or PDE5 inhibitors are ineffective. Although the class of PDE5 inhibitors represent a highly efficient treatment option in the general ED-population, 30-50% of ED patients do not respond to PDE5 inhibitors (Sabsigh et. al. 2004, De Tejada 2004) sufficiently. Clinical effectiveness of PDE5 inhibition needs at last a minimal NO signal in order to induce some turnover of cGMP, otherwise PDE5 inhibition will not be able to increase intracellular cGMP and induce vasorelaxation. I.e. the complete destruction of the cavernosal nerves during radical prostatectomy or severe diabetic neuropathies could be pathological conditions which principally limit the success of treatment with PDE5 inhibitors (De Tejada 2004). Therefore, new treatment principles within the NO/cGMP system which actively stimulate cGMP production in the corpus cavernosum (e.g. with NO-donors) are another avenue of research and might be able to overcome also PDE5-resistent ED. But also patients with mild and moderate ED will profit from stimulation and activation of the guanylate cyclase. Thus compounds that activate sGC in a NO-independent manner might therefore offer a considerable advantage over current therapies.

Two classes of compounds have been identified recently that activate the sGC NO-independently, the heme-dependent sGC stimulators, such as BAY 41-2272 according to compound of the formula (2), BAY 41-8543 according to compound of the formula (1), and BAY 63-2521 according to compound of the formula (3), and heme-independent sGC activators, such as BAY 58-2667 according to compound of the formula (5), and HMR-1766 according to compound of the formula (6), (for review see Evgenov et al., 2006).

It has been demonstrated pre-clinically that stimulation of guanylate cyclase could be used for the treatment of ED. sGC stimulators, such as YC-1 (Ko et al. 1994, Mizusawa et al. 2002), BAY 41-2272 (Stasch et al. 2001), A-350619 (Miller et al. 2003) or BAY 63-2521 (Münter et al. 2002) were pre-clinically investigated. The sGC stimulator BAY 41-2272 could relax human and rabbit cavernosal tissues *in vitro* (Kalsi et al. 2003, Baracat et al. 2003). The same compound but also BAY 63-2521 induced stable erections in rabbits after intravenous and oral applications *in vivo* (Bischoff et al. 2003; Münter 2002). In addition it was demonstrated that activation of the sGC with BAY 41-2272 could have advantages in rats with severe diabetes-induced ED in which PDE5 inhibitors were of moderate efficacy (Kalsi et al. 2004). These results show, that increase of cGMP via stimulation of sGC could result in an alternative treatment option for ED aside from PDE5 inhibitors. Since erections critically depend on Nitric Oxide (NO) and cGMP levels, 30-40% of patients do not respond to PDE5 inhibitors due to low endogenous NO/cGMP production. For such kind of PDE5 non-responders direct stimulation or activation of the soluble guanylate cyclase (sGC) by sGC stimulator might be able to overcome this lack of efficacy. However the use of these compounds - based on the ubiquitous distribution of sGC in different tissues - is clearly limited by the effects on systemic blood pressure.

We therefore investigated combinations of sGC stimulators and sGC activators, i.e. BAY 60-4552 according to compound of the formula (4), with PDE5 inhibitors, i.e. vardenafil in vivo in animal models.

We found in vivo in our animal models that:
- sGC stimulators and sGC activators, i.e. BAY 60-4552 could elicit significant erections in rabbits and rats in the range of vardenafil (Example 1).
- sGC stimulators and sGC activators, i.e. BAY 60-4552 as a stand alone treatment is effective in PDE5-resistant ED models in rabbit in which PDE5 inhibitors, i.e. vardenafil did not work (Example 1).
- sGC stimulators and sGC activators, i.e. BAY 60-4552 as a stand alone treatment produced a substantial decrease in blood pressure in rabbit and rats (Example 5) limiting the use of sGC stimulators and sGC activators as stand alone.
- combinations of sGC stimulators and sGC activators, i.e. BAY 60-4552 with PDE5 inhibitors, i.e. vardenafil is efficacious in PDE5-inhibitor-resistant ED models in rabbits (Example 2).
- combinations of sGC stimulators and sGC activators, i.e. BAY 60-4552 with PDE5 inhibitors, i.e. vardenafil showed more than additive effects in PDE5-inhibitor-resistant ED models in rabbits (Example 2).
- combinations of sGC stimulators and sGC activators, i.e. BAY 60-4552 with PDE5 inhibitors, i.e. vardenafil are efficacious in PDE5-inhibitor-resistant ED models in rats. i.e. in the L-NAME rat ED-model (Example 3) and in the Cavernous Nerve Crush rat ED-model (Example 4).
- combinations of sGC stimulators and sGC activators, i.e. BAY 60-4552 with PDE5-inhibitors, i.e. vardenafil, do not show additive or more than additive effects on blood pressure in rabbits and rats (Example 5).
- combinations of sGC stimulators and activators, i.e. BAY 60-4552 with PDE5 inhibitors, i.e. vardenafil are safe, with a hemodynamic profile similar to vardenafil (Example 5).
- combinations of sGC stimulators and sGC activators, i.e. BAY60-4552 with PDE5 inhibitors, i.e. vardenafil are efficacious in the rabbit ED model in the range of vardenafil and sildenafil on penile function in rabbits normally responding to PDE5 inhibitor, i.e. vardenafil, treatment. (Example 6)
- Combinations of sGC stimulators and sGC activators, i.e. BAY 60-4552 with PDE5 inhibitors, i.e. vardenafil have significant higher responder rates in rabbits normally responding to PDE5 inhibitors, i.e. Vardenafil, Sildenafil, Tadalafil. (Example 7)
- Clinical study (example 8)

Thus, we found completely unexpected that combinations of sGC stimulators or sGC activators with PDE5 inhibitors have not only additive but over-additive effects on penile erection in animal models in rabbits and rats. In these models PDE5 inhibitors do not work and sGC stimulators and sGC activators have only moderate efficacy.

In addition there was no overadditive effect seen on systemic blood pressure. Combinations - in contrast to sGC stimulators and sGC activators alone - showed only a modest decrease in blood-pressure. Due to the strong effects of sGC stimulators and sGC activators on systemic blood pressure this was again completely unexpected.

In addition we found that the combination avoid the haemodynamic side effects by using only a low-dose sGC stimulator component.

Finally the optimal proportions of the single components, the sGC stimulators or sGC activators and the PDE5 inhibitor in the combination could be already identified. When using similar doses of sGC stimulator or sGC activators and the PDE5 inhibitor or when reducing the sGC stimulator or sGC activator dose down to a factor of 1/10 and 1/20 of the PDE5 inhibitor dose.

The preferred dosis ranges are 0.1 mg to 1 mg of sGC stimulator or sGC activator and 2.5 to 20 mg PDE5 inhibitor.

A preferred dosis is 1 mg of sGC stimulator or sGC activator and 10 mg PDE5 inhibitor.

Another preferred dosis is 1 mg of sGC stimulator or sGC activator and 20 mg PDE5 inhibitor.

Another preferred dosis is 0.5 mg of sGC stimulator or sGC activator and 10 mg PDE5 inhibitor.

Another preferred dosis is 0.5 mg of sGC stimulator or sGC activator and 20 mg PDE5 inhibitor.

In addition we showed that combinations of sGC stimulators or sGC activators with PDE5 inhibitors have full acitivity on penile erection in animal models in which PDE5 inhibitors are already effective, however produced a substantial higher responder rate when compared to PDE5 inhibitors.

Thus the combination could have superiority to PDE5 inhibitors in the general ED population regarding treatment failures.

In summary we discovered that combinations of sGC stimulators or sGC activators with PDE5 inhibitors have not only additive but over-additive effects on penile erection in animal models in rabbits and rats but do not show this overadditive effect on blood pressure.

Due to this overadditive efficacy of combinations of sGC stimulators or sGC activators with PDE5 inhibitors, these combination are highly effective in ED in which PDE5 inhibitors work, but will produce higher first attempt responder rates.

Due to this overadditive efficacy of combinations of sGC stimulators or sGC activators with PDE5 inhibitors, these combinations are effective in ED in which PDE5 inhibitors do not work and sGC stimulators and activators alone are of moderate efficacy.

Therefore combinations of sGC stimulators or sGC activators with PDE5 inhbititors will have substantial advantages for the treatment of ED compared to the already know ED-treatment in the general ED-population, but will especially have substantial advantages in the difficult to treat ED-population.

### Disclosure of the invention

Urological disorders addressed by therapeutic agents of the invention which in particular and with substantial advantage can be treated by the above mentioned sGC stimulators or sGC activators in combination with PDE5 inhibitors, are genitourinary disorders comprising Male Sexual Dysfunction (MED).

MED is defmed by "the inability to achieve and/or maintain a penile erection for satisfactory sexual performance" (NIH Consensus Development Panel on Impotence, 1993)"

MED refers further to patients with mild, moderate and severe MED.

MED refers also to MED caused by i.e. psychogenic, organic, vascular, endocrinologic, neurogenic, arteriogenic, drug-induced, fibrotic origin.

MED refers also to ejaculatory disorders such as premature ejaculation (PE), anorgasmia (inability to achieve orgasm) or desire disorders such as hypoactive sexual desire disorder (HSDD).

In general patients with mild MED will benefit of the combination of specific sGC stimulators or sGC activators with PDE5 inhibitors and this provide advantage over methods of treatment already known in the art, i.e. PDE5 inhibitors.

In particular patients with moderate and severe MED will benefit of the combination of specific sGC stimulators or sGC activators with PDE5 inhibitors and this treatment provides substantial advantage over methods of treatment already known in the art, i.e. PDE5 inhibitors.

In addition, combinations of specific sGC stimulators or sGC activators with PDE5 inhibitors have an substantial advantage in regard to hypotensive side effects over methods of treatment already known in the art, i.e. NO-donors, sGC stimulators or sGC activators.

The invention provides sGC stimulators or sGC activators in combination whith PDE5 inhibitors which are useful for the treatment of urological disorders especially MED, and superior in efficacy over methods of treatment already known.

The invention provides sGC stimulators or sGC activators in combination whith PDE5 inhibitors which are useful for the treatment of urological disorders especially MED, and superior in the side effect profile over methods of treatment already known.

The invention provides sGC stimulators or sGC activators in combination whith PDE5 inhibitors which are useful for the treatment of urological disorders especially MED in which the sGC stimulator or sGC activator is dosed in the same range then the PDE5 inhibitor and is dosed down to 1/10 and/or 1/20 of the dose of the PDE5 inhibitor.

The invention provides sGC stimulators or sGC activators in combination whith PDE5 inhibitors which are useful for the treatment of urological disorders especially MED in which the dosis range of sGC stimulator or sGC activator is 0.1 to 1 mg and the dosis range of PDE5 inhibitor is 2.5 to 20 mg. The first study with the combined administration of a sGC stimualtor and a PDE5 inhibitor was performed in healthy male subjects (Example 8).

Guanylate cyclase (sGC) stimulator and sGC activator is preferably a compound selected from the group consisting of
- 2-[1-(2-fluorobenzyl)-1H-pyrazolo[3,4-b]pyridin-3-yl]-5-(4-morpholinyl)-4,6-pyrimidine-diamine (1), described also as example 16 in WO 00/06569,
- 2-[1-(2-fluorobenzyl)-1H-pyrazolo[3,4-b]pyridin-3-yl]-5-(4-pyridinyl)-4-pyrimidinamine (2), described also as example 1 in WO 02/42301,
- methyl-4,6-diamino-2-[1-(2-fluorobenzyl)-1H-pyrazolo[3,4-b]pyridin-3-yl]-5-pyrimidinyl-(methyl)carbamate (3), described also as example 8 in WO 03/095451,
- methyl-4,6-diamino-2-[1-(2-fluorobenzyl)-1H-pyrazolo[3,4-b]pyridin-3-yl]-5-pyrimidinyl-carbamate (4), described also as example 5 in WO 03/095451,
   and
   - 4-({(4-carboxybutyl)[2-(2-{[4-(2-phenylethyl)benzyl] oxy}phenyl)ethyl]amino}methyl) benzoic acid (5), described also as example 8a in WO 01/019780,
   and
   - 5-chloro-2-(5-chlorothiophene-2-sulfonylamino-N-(4-(morpholine-4-sulfonyl)-phenyl)-benzamide sodium salt (6), described in WO00/02851,
   - 2-(4-chloro-phenylsulfonylamino)-4,5-dimethoxy-N-(4-(thiomorpholine-4-sulfonyl)-phenyl)-benzamide (7), described in WO00/02851,

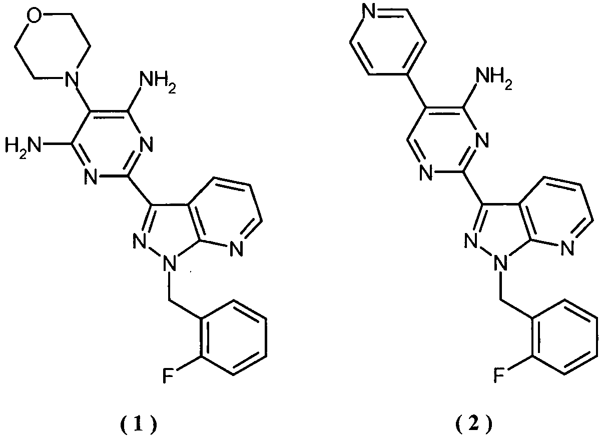

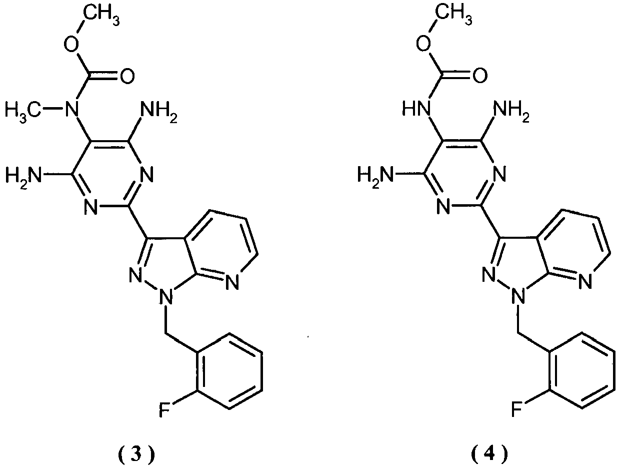

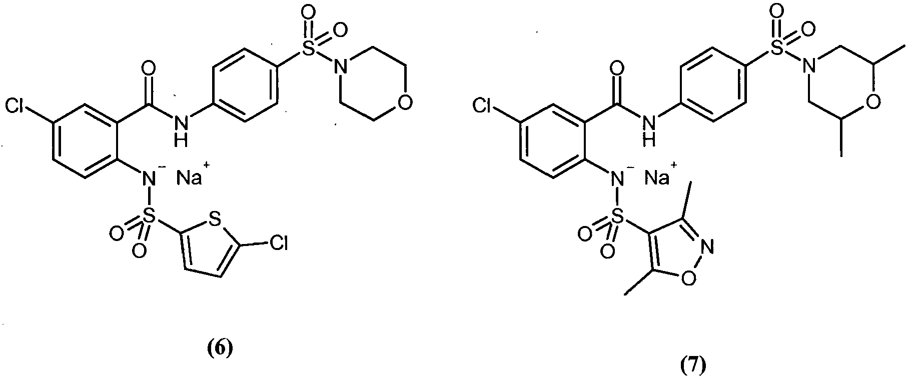

Compounds (1), (2), (3), (4), (6) and (7) are known soluble guanylate cyclase (sGC) stimulators which have been previously described for the treatment of stable angina pectoris or erectile dysfunction.

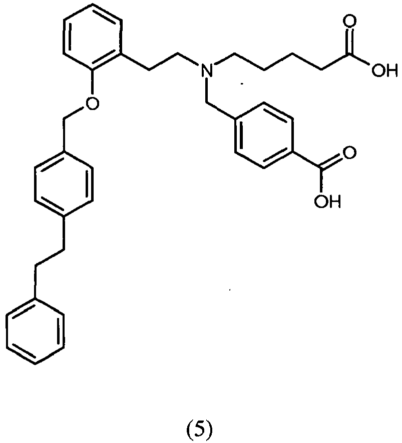

Compound (5) is known as sGC activator.

PDE-5 inhibitors which are useful for the combined treatment of urological disorders are in particular *Tadalafil* ((6R,12aR) -2,3,6,7,12,12a - Hexahydro - 2 - methyl - 6 - (3,4-methylene - dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil* (3-[2-ethoxy-5-(4-methylpiperazin-1-yl)sulfonyl-phenyl]- 7- methy 1- 9- propy 1-2,4,7,8-tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl]amino}-2-[(2S)-2-(hydroxymethyl)pyrrolidin-1-yl]-N-(pyrimidin-2-ylmethyl)pyrimidine-5-carboxamide, Mirodenafil, Lodenafil, UK 369.003, UK 371.800, *SLx 2101* of Surface Logix, *LAS 34179*Triazolo[1,2-]xanthine,6-methyl-4-propyl-2-[2-propoxy-5-(4-methylpiperazino)sulfonyl]phenyl-, or salts, hydrates or hydrates of salts of the before mentioned PDE5 inhibitors

A pharmaceutical composition of the invention is formulated to be compatible with its intended route of administration. Examples of routes of administration include parenteral e.g., intravenous, intradermal, subcutaneous' oral (e.g.' inhalation)' transdermal (topical) transmucosal and rectal administration. Pharmaceutical compositions suitable for injectable use include sterile aqueous solutions (where water soluble) or dispersions and sterile powders for the extemporaneous preparation of sterile injectable solutions or dispersions. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents for example, parabens, chlorobutanol, phenol, ascorbic acid, thimerosal, and the like. In many cases, it will be preferable to include isotonic agents, for example, sugars, polyalcohols such as maitol sorbitol sodium chloride in the composition.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or con1 starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g.' a gas such as carbon dioxide, or a nebulizer.

Systemic administration can also be by transmucosal or transderrnal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Bio degradable, biocompatible polymers can be used, such as ethylene vinyl acetate, polyanhydrides, polyglycolic acid, collagen, polyorthoesters, and polylactic acid.

In another embodiment the invention provides sGC stimulators or sGC activators in combination with PDE5 inhbitiors and their use for the preparation of pharmaceutical compositions for MED, whereby these combinations comprise either i) pharmaceutical compositions comprising a compound having a sGC stimulatory or activatory action and PDE-5 inhibitory activity, or ii) pharmaceutical compositions comprising one sGC stimulator and sGC activator and at least one PDE-5 inhibitor as a fixed combination in one application unit, or iii) a kit of parts containing at least two sets of pharmaceutical compositions, each set consisting of at least one pharmaceutical preparation comprising a PDE-5 inhibitor in units of at least one dose and at least one pharmaceutical preparation comprising a sGC activator or sGC stimulator in units of at least one dose, whereby each application unit of said pharmaceutical compositions is administered in combination, sequentially, as single dose or in multiple doses.

### In particular, the present invention provides:

A pharmaceutical composition for the treatment of a disease comprised in a group of diseases consisting of MED, especially of mild, moderate and severe MED containing
methyl-4,6-diamino-2-[1-(2-fluorobenzyl)-1H-pyrazolo[3,4-b]pyridin-3-yl]-5-pyrimidinylcarbamate (4),
and *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one) or salts, hydrates or hydrates of salts thereof.

Use of a combination of methyl-4,6-diamino-2-[1-(2-fluorobenzyl)-1H-pyrazolo[3,4-b]pyridin-3-yl]-5-pyrimidinylcarbamate (4) and Vardenafil (2-(2-Ethoxy-5-(4-ethylpiperazin-1-yl-1-sulfonyl)phenyl)-5-methyl-7-propyl-3H-imidazo (5,1-f) (1,2,4) triazin-4-one) for the preparation of a pharmaceutical composition for the treatment of a disease comprised in a group of diseases consisting of MED, especially of mild, moderate and severe MED.

A pharmaceutical composition containing methyl-4,6-diamino-2-[1-(2-fluorobenzyl)-1H-pyrazolo[3,4-b]pyridin-3-yl]-5-pyrimidinylcarbamate (4) and-Vardenafil or a salt, a hydrat or a hydrat of a salt of this PDE5 inhibitor, for the treatment of male erectile dysfunction comprising mild, moderate and severe MED.

In order to clarify the effect of sGC stimulators and sGC activators alone and in combination with vardenafil experiments are performed.

In particular sGC stimulators and sGC activators, i.e. BAY 60-4552 alone and in combination with PDE5 inhibitors, i.e. Vardenafil were tested in vivo, in 3 animal models (Example 1, 2, 3, 4) in which PDE5 inhibitors are ineffective. In addition sGC stimulators and sGC activators, i.e. BAY 60-4552 alone and in combination with PDE5 inhibitors, i.e. Vardenafil were tested in vivo on hemodynamic effects in conscious animals (Expample 5).

Combinations of sGC stimulators and sGC activators, i.e. BAY 60-4552 with PDE5 inhibitors, i.e. vardenafil are efficacious in PDE5-inhibitor-resistant ED in *in vivo* models in rabbits and rats (Example 1, 2, 3, 4, 5).

Combinations of sGC stimulators and sGC activators, i.e. BAY 60-4552 with PDE5 inhibitors, i.e. vardenafil showed more than additive effects in PDE5-inhibitor-resistant ED models *in vivo* in rabbits and rats (Example 3).

The sGC stimulators and sGC activators, i.e. BAY 60-4552 as a stand alone treatment produced a substantial decrease in blood pressure in rabbit and rats.

Combinations of sGC stimulators and sGC activators, i.e. BAY 60-4552 with PDE5-inhibitors, i.e. vardenafil, do not show additive or more than additive effects on blood pressure in rabbits and rats (i.e. Example 5).

Combinations of sGC stimulators and activators, i.e. BAY 60-4552 with PDE5 inhibitors, i.e. vardenafil are safe, with a hemodynamic profile similar to vardenafil.

The preferred embodiment of the invention is a combination of methyl-4,6-diamino-2-[1-(2-fluorobenzyl)-1H-pyrazolo[3,4-b]pyridin-3-yl]-5-pyrimidinylcarbamate (4) in which the combination comprises 0.1 to 1 mg of the sGC stiumulator or activator and 2.5 to 20 mg of the PDE5 inhibitor.

Another preferred embodiment of the invention is a combination according to claims 1 for the use as a medicament.

Another preferred embodiment of the invention is the use of a combination as disclosed above for the manufacture fo a medicament for the treatment of Male Sexual Dysfuntion (MED).

Another preferred embodiment of the invention is a combination as disclosed above for the use in Sexual Dysfuntion (MED).

Another preferred embodiment of the invention a the use of a combination as disclosed above for the manufacture fo a medicament for the treatment of Male Sexual Dysfuntion (MED) in cases where the patient suffers form a PDE5 inhibitor resistance.

Another preferred embodiment of the invention is a combination as disclosed above for the use in Male Sexual Dysfuntion (MED) in cases where the patient suffers form a PDE5 inhibitor resistance.

Another preferred embodiment of the invention is a pharmaceutical formulation comprising at least one combination as disclosed above.

Another preferred embodiment of the invention is a pharmaceutical formulation comprising at least one combination as disclosed above for the use in Male Sexual Dysfuntion (MED).

Another preferred embodiment of the invention is a pharmaceutical formulation comprising at least one combination as disclosed above for the use in Male Sexual Dysfuntion (MED) in cases where the patient suffers form a PDE5 inhibitor resistance.

### Experimental Part:

### Example 1

The conscious rabbit ED model previously described (Bischoff and Schneider 2001) was used for the investigation of Erectile function. In brief: conscious male chinchilla rabbits (3-4kg) were orally treated with the test compounds or with vehicle via gavage, followed by i.v. injection of the NO-donnor sodium nitro-prusside (SNP) in the ear vein, 90 minutes after oral applications. The SNP injections were done in a high dose setting (0.2mg/kg SNP i.v) and a low dose setting (0.02mg/kg SNP i.v.) corresponding to the general ED-population and the PDE5 non-responder ED-population, respectively. Penile length was quantified in 5 minutes intervals after injection of the SNP. The rectile function was measured after oral application of the test compounds, i.e. vehicle, PDE5 inhibitors, i.e. vardenafil, sGC stimulators, i.e. BAY 60-4552. or combinations of PDE5 inhibitors, i.e. vardenafil with sGC stimulators i.e. BAY 60-4552.

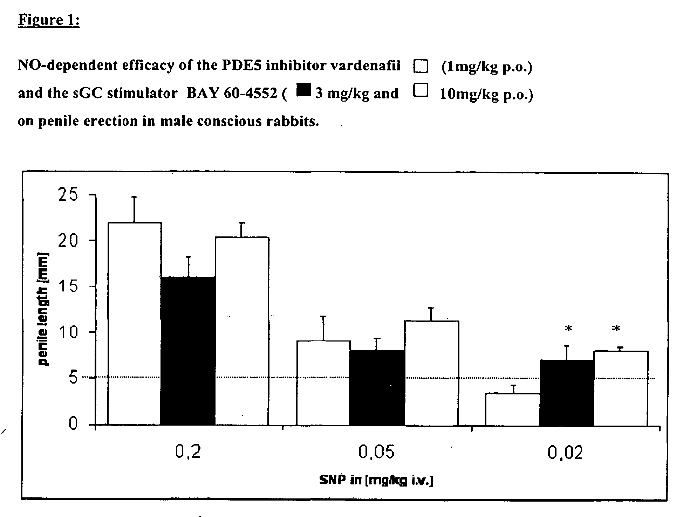

In order to reflect the low NO/cGMP production in PDE5-resistant ED population the dose of SNP injected into the ear vein 90 minutes after application of the test compounds, was 10-fold reduced to 0.02mg/kg SNP i.v.. In contrast to the previous experiment with high SNP dose, vardenafil like the other PDE5 inhibitors sildenafil and tadalafil (not shown) were not able to produce any physiologically relevant erection. In contrast 3 and 10mg/kg BAY 60-4552 were able to induce physiologically relevant erections in the rabbits although NO-supply is impaired. These results are demonstrating that the sGC stimulator BAY 60-4552 could overcome low-NO/cGMP conditions and might be useful for the treatment of PDE5-resistant ED. (Figure 1)

### Example 2

The combination of 1mg/kg Vardenafil - which is not effective in the aforementioned model - with 3mg/kg BAY 60-4552 was tested in the low SNP model as described in Example 1. This and the other tested combinations of the sGC stimulator with vardenafil produces overadditive effects on penile erection. Dose-dependent penile erection were induced when combining vardenafil 0.3 mg/kg with 3, 1, 0.3. mg/kg BAY 60-4552 (Figure 2a). In addition, dose-dependent penile erections were induced when combining vardenafil 1 mg/kg with 3, 1, 0.3. and 0.1 mg/kg BAY 60-4552 (Figure 2b). The FDC containing 1mg/kg vardenafil and 0.1 mg/kg BAY 60-4552 produced still relevant erections which were higher than that of BAY 60-4552 as a stand alone treatment (not shown). These data indicate the overadditive effects of combinations of the sGC stimulator i.e. BAY 60-4552 and the PDE5 inhibitor i.e Vardenafil on penile function. These results also indicate the superiority over PDE5 inhibitor or sGC stimulator or sGC activator stand-alone treatment therapy.

### Example 3

Intracavernosal pressure reflectiong penile erection was measured as described previously (Giuliano et al. 1993, Sandner 2008b). In brief: Male Wistar Rats (150-250g) were anaesthetized with isolfurane, after laparotomy a pressure catheter is implanted in the corpus cavernosum and the cavernous nerve is carefully prepared for electric field stimulation (EFS). The intracavernous pressure is registered via a pressure transducer (MLT0698) and amplified and stored with the PowerLab® system. For induction of ED and application of test compounds a venous catheter is implanted in the femoral vein. The injection of L-NAME (3mg/kg bolus i.v) blocking the NO synthases was performed to induce ED. 10 Minutes after injection of L-NAME test compounds (Placebo, BAY 60-4552, Vardenafil and the combination) were applied.

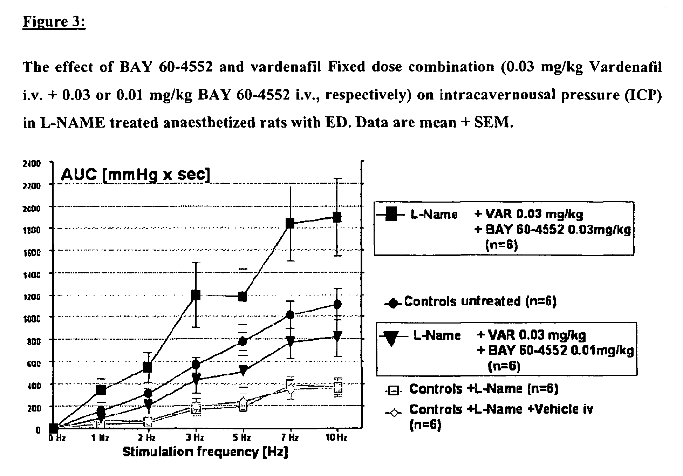

In a first series of experiments L-NAME was able to block elevation of the ICP. the FDC with 10)µg/kg BAY 60-4552 produced a significant effect when compared to the L-NAME-vehicle group. The 30µg/kg combination showed an overadditive effect and increased the AUC derived from the ICP over time more than seen in the non L-NAME treated controls (Figure 3). These data demonstrated that combinations of BAY 60-4552 and vardenafil are very effective in the L-NAME ED-model and were able to completely restore erectile function in a disease model reflecting also the PDE5 non-responder population. These data further indicate the substantial advantage of the aforementioned combinations over current treatment options for ED in this difficult to treat patients.

### Example 4

Patients after radical prostatectomy do not respond to PDE5 inhibitors since the cavernous nerves are at least partly destroyed and removed during surgery resulting in the inability to produce NO and induce cGMP elevation in the corpus cavernosum. This nerve crush could be mimicked in anaesthetized rats generating an ED model for the difficult to treat ED-population not, or not fully, responding to PDE5 inhibitor treatment.

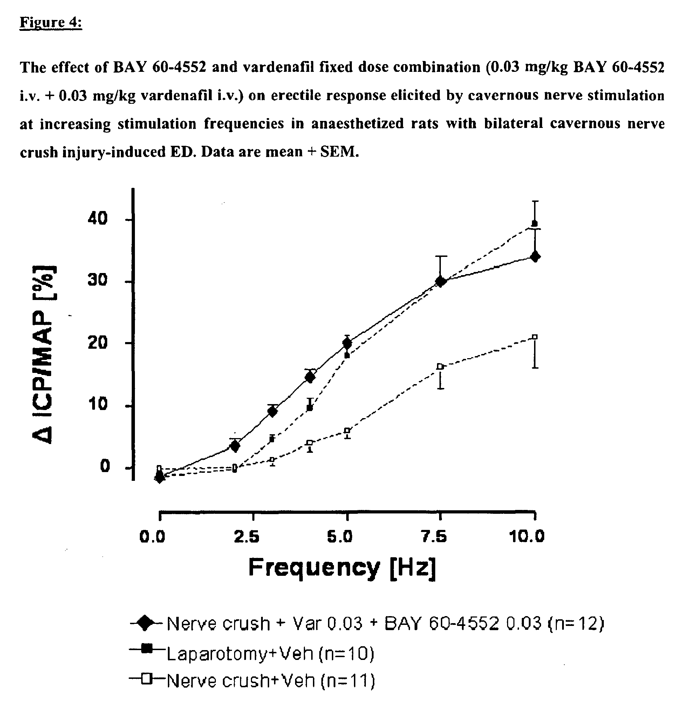

Bilateral cavernous nerve crush injury resulted in a significant erectile dysfunction if compared to SHAM operated animals. The application of a combination of a PDE5 inhibitor i.e. vardenafil with a sGC stimulator i.e. BAY 60-4552 resulted in a full recovery of these animals and the ICP was not significantly different from the SHAM operated group. Within this model the same dose of the PDE5 inhibitor i.e. 30µg/kg Vardenafil with the sGC stimulator i.e. 30µg/kg BAY 60-4552 produced complete recovery (Figure 4).These data indicate that the combination of a PDE5 inhibitor, i.e. vardenafil with a sGC stimulator or and sGC activator, i.e. BAY 60-4552 is able to induce significant erections also after cavernous nerve damage. These data further indicate the substantial advantage of the aforementioned combinations over current treatment options for ED in this difficult to treat patients i.e. of patients after prostatectomy.

### Example 5

The haemodynamic effects of vardenafil, BAY 60-4552 alone and in combination were carefully analyzed in conscious male chinchilla rabbits as described previously (Sandner 2008a). In brief, telemetric implants (C50 PXT-DSI®) were used. Signals were received with RMC1-DSI® receiver plates, compiled and analyzied with PONEMAH® physiology platform software.

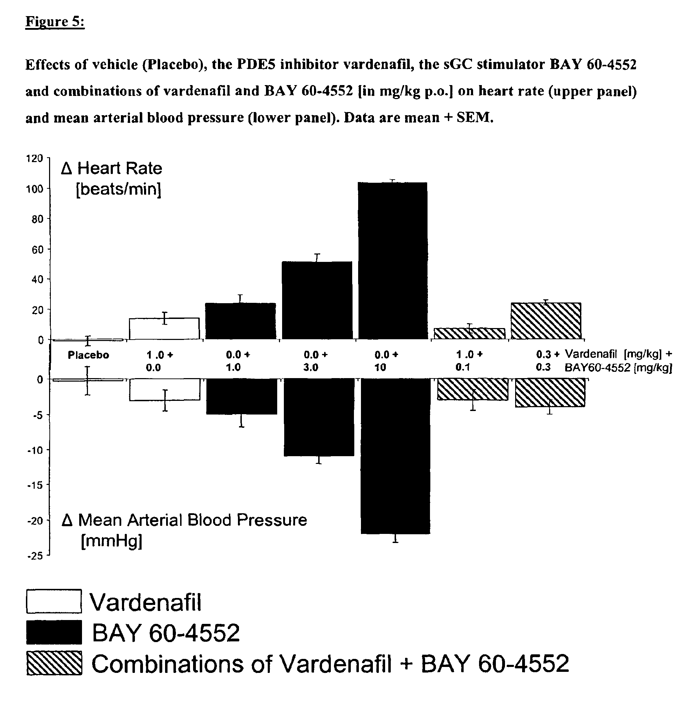

Application of a PDE5 inhibitor i.e. Vardenafil (1mg/kg p.o.) produced a low decrease in mean arterial blood pressure (-4mmHg) accompanied by an increase in heart rat (+14 bpm). The sGC stimulator, i.e. BAY 60-4552 stand-alone dose dependently produced a decrease in blood pressure of -5, -11, -22 mmHg in the 1, 3 and 10mg/kg dose respectively, which was accompanied by heart rate increase of +24, +51 and +103 bpm. The combination the PDE5 inhibitor, i.e. vardenafil with the sGC stimulator, i.e. BAY 60-4552 produced a blood pressure decrease of -3 and -4 mmHg and heart rate increase of +7 and +24 bpm in the combination of of 1+0.1 and 0.3+0.3 mg/kg respectively. Thus the increase in efficacy of the FDC in ED function was not accompanied by haemdoynamic side effects compared with vardenafil (Figure 5).

### Example 6

The conscious rabbit ED model previously described (Bischoff and Schneider 2001) was used for the investigation of Erectile function as described in Example 1. The SNP injections were done in a high dose setting (0.2mg/kg SNP i.v) corresponding to the general ED-population in which PDE5 inhibitors are fully active. Penile length was quantified in 5 minutes intervals after injection of the SNP. The erectile function was measured after oral application of the test compounds, i.e. vehicle, PDE5 inhibitors, i.e. vardenafil, or combinations of PDE5 inhibitors, i.e. vardenafil with sGC stimulators, i.e. BAY 60-4552.

PDE5 inhibitors, i.e. vardenafil and combinations of PDE5inhibitors and sGC stimulators or activators, i.e. vardenafil + BAY 60-4552 showed a similar range of efficacy which was on the upper-end of the effects which could be seen in these experiments.

### Example 7

The conscious rabbit ED model as described in Expample 1 and Example 6 was used for assessment of responderrates. The SNP injections were done in a high dose setting (0.2mg/kg SNP i.v) corresponding to the general ED-population in which PDE5 inhibitors are fully active. Penile length was quantified in 5 minutes intervals after injection of the SNP. "Non response" was defined as penile length ≤ 5 mm during the whole observation perios after the SNP injection. Penile length was quantified after treatment with PDE5 inhibitors, i.e. vardenafil, sildenafil, tadalafil, or the combination of an sGC stimulator or sGC activator, i.e. BAY 60-4552 with and PDE5 inhbitior, i.e. vardenafil.

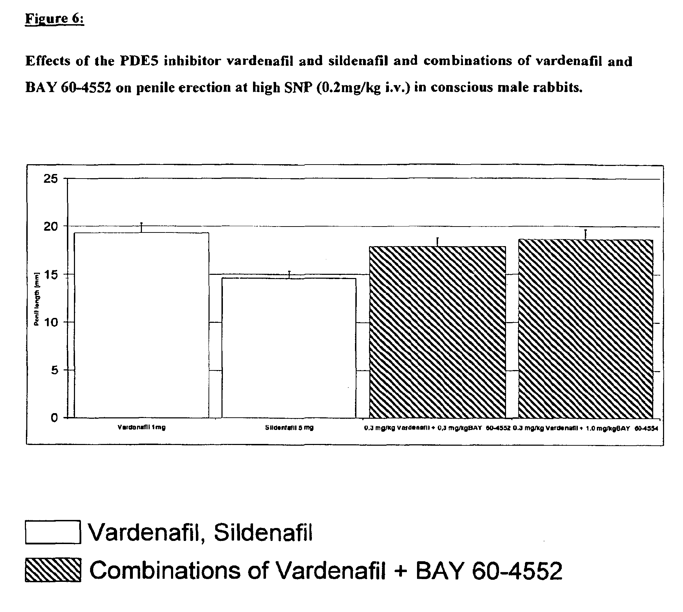

When counting first-time failures in the rabbit-ED model at high SNP - in which PDE5 inhibitor induced significant erections (Figure 6) - significant higher rates of responders were identified in the groups receiving the combination therapy. (Table 1).

### Example 8

One phase I study (#14441) was performed to investigate the influence of co-administration of different single doses of the PDE5 inhibitor 'vardenafil' (tablet formulation) on safety, tolerability, pharmacokinetics and pharmacodynamics of single oral doses of the sGC stimulator 'BAY 60-4552' (oral solution) in healthy male subjects. The study was designed as a randomized, single-blind, placebo-controlled, group-comparison, dose-escalation study. In 5 dose steps (DS) 1.0 mg BAY 60-4552 was co-administered with 2.5 mg (DS 1), 5.0 mg (DS 2), 10 mg (DS 3) and 20 mg vardenafil (DS 4) and in DS 5, 2.0 mg BAY 60-4552 was co-administered with 20 mg vardenafil. Each dose step was performed with 9 healthy male subjects treated with the combination of vardenafil and BAY 60-4552 and 3 were treated with placebos, respectively.

To date, maximum co-administered doses were 2.0 mg BAY 60-4552 and 20 mg vardenafil. All 5 dose steps were safe and well tolerated. There was no death. All adverse events were of mild or moderate intensity. Most frequent treatment emergent drug-related adverse events were headache, flush, spontaneous erection, nasal congestion, tiredness, orthostatic reaction and sensation of pressure in eyes. All these adverse events were reported in previous studies performed with vardenafil or BAY 60-4552.

After the co-administration of BAY 60-4552 and vardenafil, 1 to 2 spontaneous erections were observed in each dose step. No spontaneous erection was observed after placebo treatment. The incidence of spontaneous erection was not dose-related, neither to vardenafil nor to BAY 60-4552. Compared to placebo, the Δ heart rate (1 h after drug intake) was increased by about 10 beats/min independent of the administered BAY 60-4552 or vardenafil dose (figure a). The effect on diastolic blood presseure was depicted in figure b) and mainly related to BAY 60-4552 dosages. Figure c shows the changes of the cGMP levels. The range of changes in vital signs observed after co-administration of the sGC stimulator and the PDE5 inhibitor were less or similar when 5mg, 10 mg or 20 mg vardenafil was given alone (see study #0094).

There were no clinically relevant changes on electrocardiogram (ECG), heart rate, blood pressure and clinical laboratory parameters, hematology and urinalysis.

Vardenafil in combination with 1 mg or 2 mg BAY 60-4552 demonstrated dose-proportional AUC and Cmax in the range 2.5 mg to 10 mg, while slightly higher than proportional exposure was observed at the 20 mg vardenafil level. Vardenafil was rapidly absorbed (median tmax of 0.75 to 1 h) and eliminated with a half-life of 3 to 5 h. BAY 60-4552 exposure increased in proportion to the dose following administration of 1 mg to 2 mg doses in combination with vardenafil (2.5 mg to 20 mg). BAY 60-4552 reached maximum plasma concentrations after 0.5 to 1 h (median tmax) and was eliminated with a half-life of 13 to 14 h. The exposure of vardenafil and BAY 60-4552 following concomitant administration was consistent with historical data collected after administration of each compound alone. This supports the expectation of no pharmacokinetic interaction between both compounds based on the fact that neither vardenafil nor BAY 60-4552 is an inhibitor or inducer of the involved metabolic enzymes. The pharmacokinetics of BAY 60-4552 are time linear.

Previous studies have shown that the separate and single administration of 2.0 mg BAY 60-4552 and the single administration of 20 mg vardenafil, respectively, had no clinically relevant effect on heart rate and blood pressure. Based on preclinical data for co-administering BAY 60-4552 and vardenafil (rats, rabbits), even at higher doses, effects on the cardiovascular system are expected to be low and not clinically relevant.

### References:

Baracat JS, Teixeira CE, Okuyama CE et al. (2003): Relaxing effects induced by the soluble guanylyl cyclase stimulator BAY 41-2272 in human and rabbit corpus cavernosum. Eur. J. Pharmacol 477(2):163-169
Bischoff E, Schneider K. (2001): A conscious-rabbit model to study vardenafil hydrochloride and other agents that influence penile erection. Int. J. Impot. Res. 13: 230-235
Bischoff E, Schramm M, Straub A et al. (2003): BAY 41-2272: a stimulator of soluble guanylyl cyclase induces nitric oxide-dependent penile erection in vivo. Urology 61:464-467.
De Tejada IS (2004) Therapeutic strategies for optimizing PDE5 inhibitor therapy in patients with erectile dysfunction considered difficult or challenging to treat. Int J Impot Res 216:40-42
Evgenov OV, Pacher P, Schmidt PM et al. (2006) NO-independent stimulators and activators of soluble guanylate cyclase: discovery and therapeutic potential. Nat Rev Drug Discov 5(9):755-68
Giuliano F, Bernabe J, Jardin A et al. (1993): Antierectile role of the sympathetic nervous system in rats. J. Urol. 150:519-524
Kalsi JS, Ralph DJ, Madge DJ et al. (2004): A comparative study of sildenafil, NCX-911 and BAY41-2272 on the anococcygeus muscle of diabetic rats. Int. J. Impot. Res. 16:479-485
Ko FN, Wu CC, Kuo SC et al. (1994) YC-1, a novel activator of platelet guanylate cyclase. Blood 84(12):4226-4233
Köhler TS and McVary KT. (2008): The Relationship between Erectile Dysfunction and Lower Urinary Tract Symptoms and the Role of Phosphodiesterase Type 5 Inhibitors. Eur. Urol. 2008 Sep 4. [Epub ahead of print]
Lue TF (2000) Erectile dysfunction. New Eng. J. Med. 342:1802-1813
Miller LN, Nakane M, Hsieh GC et al. (2003): A-350619: a novel activator of soluble guanylyl cyclase. Life Sci. 72(9):1015-1025
Mizusawa H, Hedlund P, Brioni JD et al. (2002): Nitric oxide independent activation of guanylate cyclase by YC-1 causes erectile response in rat. J. Urol. 167(5):2276-2281
Münter K, Weigand S, Bischoff E. (2002): BAY 63-2552: Its Effect on Penile Erections in Concious Rabbits. BSP-Pharma Report PH 32390
Porst H, Sandner P, Ulbrich E. (2008): Vardenafil in the treatment of lower urinary tract symptoms secondary to benign prostatic hyperplasia. Curr. Urol. Rep. 9:295-301.
Sandner P, Hütter J, Tinel H et al. (2007a): PDE5 inhibitors beyond erectile dysfunction. Int. J. Impot. Res. 19(6):533-543
Sandner P. (2008a): Efficacy and safety of the sGC stimulator BAY 60-4552 alone and in combination with the PDE5 inhibitor vardenafil (BAY 38-9456) for the treatment of PDE5-resistant Erectile Dysfunction. (BSP-Pharma-report in preparation)
Sandner P. (2008b): Efficacy of the fixed dose combination of the sGC stimulator BAY 69-4552 in combination with the PDE5 inhibitor vardenafil (BAY 38-9456) in rat ED models. (BSP-Pharma-report in preparation)
Sandner P., Svenstrup N., Tinel H. et al. (2007b): PDE5 inhibitors and erectile dysfunction (ED). Expert Opin. Ther. Patents 18:21-33
Schultheiss D and Stief CG (1999) Physiology and pathophysiology of erection: consequences for present medical therapy of erectile dysfunction. Andrologia 31(Suppl 1):59-64
Shabsigh R (2004): Therapy of ED: PDE-5 Inhibitors. Endocrine. 23:135-141.
Shabsigh R, Seftel AD, Rosen RC et al.(2006):Review of time of onset and duration of clinical efficacy of phosphodiesterase type 5 inhibitors in treatment of erectile dysfunction. Urology.68:689-696
Stasch JP, Becker EM, Alonso-Alija C et al. (2001): NO-independent regulatory site on soluble guanylate cyclase. Nature 8:212-215

Table 1:Number of animals non-responding to the PDE5 inhibitors vardenafil, sildenafil, tadalafil and to combinations in the high SNP (0.2mg/kg i.v.) rabbit ED-model.
Figure 1: NO-dependent efficacy of the PDE5 inhibitor vardenafil (1mg/kg p.o.) and the sGC stimulator BAY 60-4552 (3 mg/kg and 10mg/kg p.o.) on penile erection in male conscious rabbits.
Figure 2A: Effects of PDE5 inhibitor vardenafil, sildenafil, tadalafil, the sGC stimulator BAY 60-4552 and combinations of vardenafil and BAY 60-4552 penile erection at low SNP (0.02mg/kg i.v.) in conscious male rabbits.
Figure 2B: Effects of PDE5 inhibitor vardenafil, sildenafil, tadalafil, the sGC stimulator BAY 60-4552 and combinations of vardenafil and BAY 60-4552 penile erection at low SNP (0.02mg/kg i.v.) in conscious male rabbits.
Figure 3: The effect of BAY 60-4552 and vardenafil Fixed dose combination (0.03 mg/kg Vardenafil i.v. + 0.03 or 0.01 mg/kg BAY 60-4552 i.v., respectively) on intracavernousal pressure (ICP) in L-NAME treated anaesthetized rats with ED. Data are mean + SEM.
Figure 4: The effect of BAY 60-4552 and vardenafil fixed dose combination (0.03 mg/kg BAY 60-4552 i.v. + 0.03 mg/kg vardenafil i.v.) on erectile response elicited by cavernous nerve stimulation at increasing stimulation frequencies in anaesthetized rats with bilateral cavernous nerve crush injury-induced ED. Data are mean + SEM.
Figure 5: Effects of vehicle (Placebo), the PDE5 inhibitor vardenafil, the sGC stimulator BAY 60-4552 and combinations of vardenafil and BAY 60-4552 [in mg/kg p.o.] on heart rate (upper panel) and mean arterial blood pressure (lower panel). Data are mean + SEM.
Figure 6: Effects of the PDE5 inhibitor vardenafil and sildenafil and combinations of vardenafil and BAY 60-4552 on penile erection at high SNP (0.2mg/kg i.v.) in conscious male rabbits.

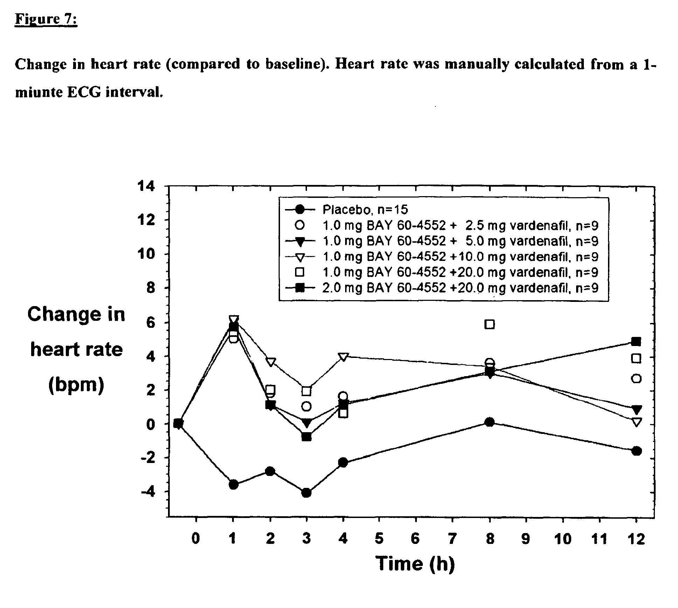

Figure 7: Change in heart rate (compared to baseline). Heart rate was manually calculated from a 1-miunte ECG interval.

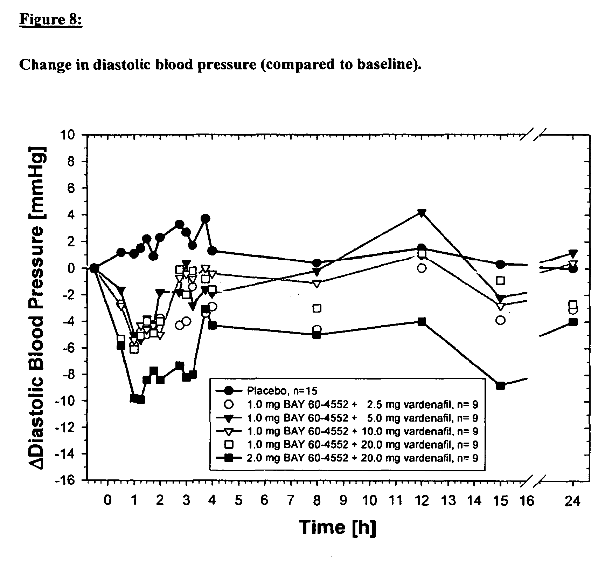

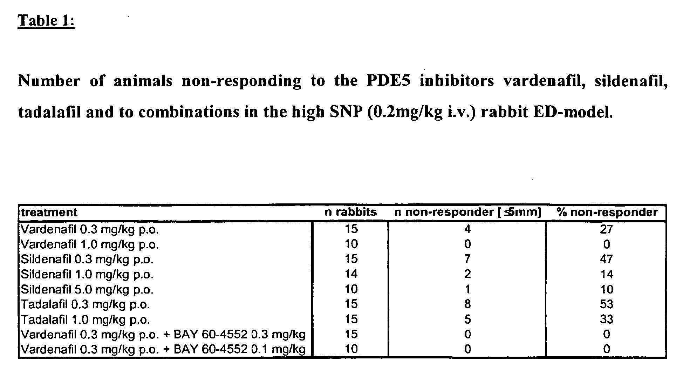

Figure 8: Change in diastolic blood pressure (compared to baseline).

## Claims

1. Combination of methyl-4,6-diamino-2-[1-(2-fluorobenzyl)-1H-pyrazolo[3,4-b]pyridin-3-yl]-5-pyrimidinylcarbamate (4) and vardenafil.

2. Use of combination according to claim 1 for the manufacture of a medicament for use in the treatment of Male Sexual Dysfuntion (MED) in cases where the patient suffers form a PDE5 inhibitor resistance.

3. Combination according to claim 1 for the use in the treatment of Male Sexual Dysfuntion (MED) in cases where the patient suffers form a PDE5 inhibitor resistance.

4. Pharmaceutical formulation comprising the combination according to claim 1.

5. Pharmaceutical formulation comprising the combination according to claim 1 for the use in treating Male Sexual Dysfuntion (MED) in cases where the patient suffers form a PDE5 inhibitor resistance.

## Patentansprüche

1. Kombination von 4,6-Diamino-2-[1-(2-fluorbenzyl)-1H-pyrazolo[3,4-b]pyridin-3-yl]-5-pyrimidinylcarbamidsäuremethylester(4) und Vardenafil.

2. Verwendung einer Kombination nach Anspruch 1 zur Herstellung eines Medikaments zur Verwendung bei der Behandlung von männlicher sexueller Dysfunktion (Male Sexual Dysfunction, MED) in Fällen, bei denen der Patient an einer PDE5-Inhibitor-Resistenz leidet.

3. Kombination nach Anspruch 1 zur Verwendung bei der Behandlung von männlicher sexueller Dysfunktion (Male Sexual Dysfunction, MED) in Fällen, bei denen der Patient an einer PDE5-Inhibitor-Resistenz leidet.

4. Pharmazeutische Formulierung, umfassend die Kombination nach Anspruch 1.

5. Pharmazeutische Formulierung, umfassend die Kombination nach Anspruch 1, zur Verwendung bei der Behandlung von männlicher sexueller Dysfunktion (Male Sexual Dysfunction, MED) in Fällen, bei denen der Patient an einer PDE5-Inhibitor-Resistenz leidet.

## Revendications

1. Combinaison de 4,6-diamino-2-[1-(2-fluorobenzyl)- 1H-pyrazolo[3,4-b]pyridin-3-yl]-5-pyrimidinylcarbamate de méthyle (4) et de vardénafil.

2. Utilisation de la combinaison selon la revendication 1, pour l'élaboration d'un médicament destiné à une utilisation dans le traitement de la Dysfonction Sexuelle Masculine (MED) dans les cas où le patient souffre d'une résistance à l'inhibiteur de PDE5.

3. Combinaison selon la revendication 1, pour une utilisation dans le traitement de la Dysfonction Sexuelle Masculine (MED) dans les cas où le patient souffre d'une résistance à l'inhibiteur de PDE5.

4. Formulation pharmaceutique comprenant la combinaison selon la revendication 1.

5. Formulation pharmaceutique comprenant la combinaison selon la revendication 1, pour une utilisation dans le traitement de la Dysfonction Sexuelle Masculine (MED) dans les cas où le patient souffre d'une résistance à l'inhibiteur de PDE5.
